# EUROPEAN PATENT APPLICATION

(11) **EP 2 479 547 A1**
(43) Date of publication of application: **25.07.2012**
(21) Application number: 10817098.6
(22) Date of filing: 09.09.2010
(51) Int. Cl.: G01L 5/04, A61M 25/02, G09B 19/00

(54) **MEASUREMENT DEVICE, MEDICAL DEVICE, TRAINING DEVICE, AND MEASUREMENT METHOD**

(30) Priority: 15.09.2009 JP 2009213369
(71) Applicant: National University Corporation Nagoya Institute of Technology, Showa-ku Nagoya-shi Aichi 466-0061 (JP); National University Corporation Nagoya University, Aichi 464-8601 (JP); NTN Corporation, Osaka-shi, Osaka 550-0003 (JP)
(72) Inventor: FUJIMOTO, Hideo, Nagoya-shi Aichi 466-0061 (JP); MIYACHI, Shigeru, Nagoya-shi Aichi 464-8601 (JP); NAGANO, Yoshitaka, Iwata-shi Shizuoka 438-8510 (JP); OZAKI, Takayoshi, Iwata-shi Shizuoka 438-8510 (JP)
(74) Representative: Bockhorni, Josef
(86) International application number: PCT/JP2010/065522
(87) International publication number: WO 2011/033985

(57) **Abstract**

This measurement device is a measurement device for measuring compressive force and pulling force in a direction of a longitudinal axis applied to a linear body (1) having flexibility, and it includes a main body (2) in which a through hole (3) for inserting the linear body is formed. The through hole (3) is formed to allow bending of the linear body (1) in an arc shape in the through hole (3) and variation in degree of bending of the linear body (1) in accordance with compressive force and pulling force. This measurement device further includes a sensor (30) for detecting a degree of bending of the linear body (1) and a conversion circuit 12 for converting the degree of bending detected by the sensor (30) into a signal indicating compressive force and pulling force applied to the linear body (1).

## Description

### TECHNICAL FIELD

This invention relates to a measurement device, a medical device, a training device, and a measurement method, and particularly to a measurement device for measuring force applied to a linear body having flexibility, a medical device, a training device, and a measurement method.

### BACKGROUND ART

A linear body having flexibility has been put into practical use as a linear medical appliance inserted in a vessel in a body. For example, a guide wire or a catheter inserted in a vessel in a body such as a blood vessel, a ureter, a bronchus, an alimentary canal, or a lymph vessel, or a wire having an embolus coil attached at a tip end for embolizing an aneurysm has been known. Such a linear body is inserted in a vessel in a body and guided to a destination through an operation from outside the body.

In many cases, the vessel in which the linear body is inserted is not necessarily linear but partially flexed or branched. In addition, a diameter of the vessel is not necessarily uniform, and the vessel itself may become thinner or a diameter of the vessel may be made smaller by an obstacle located in the vessel such as a thrombus in a blood vessel. A conventional linear body, however, has not been provided with means for sensing a condition in a direction of travel of the linear body, and it has been necessary to use operator's intuition in operating the linear body and the operator has had to be skilled in the operation for guiding the linear body from outside the body. A device provided with a pressure sensor at a tip end of a linear body is disclosed as a device sensing presence of an obstacle in a direction of travel of the linear body (see, for example, PTL 1 (Japanese Patent Laying-Open No. 10-263089)).

On the other hand, it is difficult to realize a device provided with a pressure sensor at the tip end of a linear body, in particular when the linear body is extremely thin. For example, a guide wire to be inserted in a cerebral blood vessel has a diameter around 0.35 mm, and it is difficult to provide a small pressure sensor at the tip end of such an extremely thin linear body. In addition, it is more difficult to insert a wire into the linear body in order to extract a signal from the pressure sensor to the outside.

Moreover, if the vessel in which the linear body is inserted is flexed or if a diameter of the vessel is small, insertion resistance of the linear body is affected by friction with the vessel. Accordingly, an output from the pressure sensor provided at the tip end of the linear body may not necessarily be in agreement with kinesthetic sense of the operator at the time of insertion. Therefore, even when the device provided with the pressure sensor at the tip end of the linear body is used, the operator operates the linear body based on kinesthetic sense information of the insertion resistance of the linear body externally held with fingers of the operator, that is, relying on intuition of the operator. Further, as it is only the operator that can feel the kinesthetic sense, it is difficult to quantify manipulation of a skilled operator so as to transfer the skill to a less experienced operator.

In addition, it is not cost effective to prepare linear bodies of various shapes and materials for adaptation to different applications and to provide pressure sensors in respective linear bodies, and manufacturing cost is increased.

As a technique for solving such problems, for example, PTL 2 (Japanese Patent Laying-Open No. 2007-292711) discloses a construction as follows. Namely, a measurement device for measuring compressive force in a direction of a longitudinal axis applied to a linear body having flexibility includes a main body in which a through hole through which the linear body passes is formed, the linear body bending in a prescribed direction within the through hole when the compressive force is applied to the linear body, a sensor for detecting the degree of bending, and a conversion circuit for converting the detected degree of bending into the compressive force applied to the linear body.

The constructions described in PTLs 1 and 2 are for measuring compressive force applied to the linear body. Meanwhile, as a technique for measuring pulling force applied to a linear body, for example, PTL 3 (Japanese Patent Laying-Open No. 2002-90239) discloses a construction as follows. Namely, a linear body tension detection device for detecting pulling force applied to a linear body includes an attachment portion freely attachable to/removable from a support at a portion intermediate in the linear body with movement of the linear body being allowed, a flexure portion provided in a central portion of the attachment portion and projecting in a direction orthogonal to the linear body, for flexing the linear body, and a detector for detecting force applied by the linear body when the linear body flexed by this flexure portion returns to a straight shape, so that pulling force is detected based on force detected by the detector.

### CITATION LIST

### PATENT LITERATURE

PTL 1: Japanese Patent Laying-Open No. 10-263089
PTL 2: Japanese Patent Laying-Open No. 2007-292711
PTL 3: Japanese Patent Laying-Open No. 2002-90239

### SUMMARY OF INVENTION

### TECHNICAL PROBLEM

The constructions described in PTLs 1 to 3, however, can detect only one of compressive force and pulling force on the linear body.

Measurement of compressive force applied to the linear body is a function necessary for avoiding damage to a human body as a result of application of excessive compressive force, that is, insertion force, to the linear body introduced in a body.

In addition, in embolization of a cerebral aneurysm with a coil, in feeding a treatment device into a body through a linear body, alignment of the treatment device or change of the treatment device to another device of a different size for better adaptation may be made. In this case, an operation for pulling back the linear body is performed. If the linear body cannot smoothly be pulled back, however, it is expected that the treatment device or the linear body inserted in the body is caught in the body. If the linear body is forcibly pulled back in such a case, excessive load will be applied to the human body, the treatment device, and the linear body, which is not preferred.

This invention was made to solve the above-described problems, and an object of the invention is to provide a measurement device, a medical device, a training device, and a measurement method, with which a linear body can satisfactorily be operated with a simplified construction.

### SOLUTION TO PROBLEM

In order to solve the problems above, a measurement device according to one aspect of this invention is a measurement device for measuring compressive force and pulling force in a direction of a longitudinal axis applied to a linear body having flexibility, it includes a main body in which a through hole for inserting the linear body is formed, the through hole being formed to allow bending of the linear body in an arc shape in the through hole and variation in degree of bending of the linear body in accordance with the compressive force and the pulling force, a sensor for detecting a degree of bending of the linear body, and a conversion circuit for converting the degree of bending detected by the sensor into a signal indicating compressive force and pulling force applied to the linear body, and the conversion circuit converts the detected degree of bending into a signal indicating compressive force applied to the linear body when the degree of bending of the linear body increases as compared with the degree of bending of the linear body while compressive force and pulling force are not applied to the linear body and converts the detected degree of bending into a signal indicating pulling force applied to the linear body when the degree of bending of the linear body decreases as compared with the degree of bending of the linear body while compressive force and pulling force are not applied to the linear body.

Preferably, the sensor detects a position of the linear body in the through hole, and the conversion circuit converts an amount of displacement from a reference position of the linear body while compressive force and pulling force are not applied to the linear body into a signal indicating compressive force applied to the linear body when the linear body is displaced from the reference position toward an outer periphery and converts an amount of displacement from the reference position into a signal indicating pulling force applied to the linear body when the linear body is displaced from the reference position toward an inner periphery.

Preferably, the through hole has a first end portion and a second end portion, a first restraint portion and a second restraint portion provided at the first end portion and the second end portion respectively, in a manner adjacent thereto, for restricting movement of the linear body in a direction other than the direction of the longitudinal axis, and a detection portion provided between the first restraint portion and the second restraint portion, formed such that the linear body is bent in an arc shape, and expanded in a direction of an inner periphery and a direction of an outer periphery of the linear body bent in the arc shape.

Further preferably, the through hole further has a groove portion provided between the first restraint portion and the second restraint portion and connected to the detection portion, and being greater in length in a direction at a right angle to a direction of insertion of the linear body and a direction of radius of the linear body bent in the arc shape than the detection portion.

Further preferably, the groove portion is connected to an end portion of the detection portion on an inner periphery side of the linear body bent in the arc shape.

Preferably, the sensor is attachable to/removable from the main body, the main body is provided with a marker, and the sensor detects a position of the marker while it is attached to the main body and corrects the detected degree of bending of the linear body based on the detected position of the marker.

Preferably, the sensor is an optical line sensor including a light receiver receiving light emitted by a light source, for detecting the degree of bending of the linear body by detecting a position at which a quantity of light received by the light receiver decreases as the linear body cuts off the light emitted by the light source.

Preferably, the measurement device includes a plurality of the sensors, and the conversion circuit converts the degree of bending detected by the plurality of sensors into compressive force and pulling force applied to the linear body.

Preferably, the measurement device includes at least one of a visualizing instrument for displaying compressive force and pulling force resulting from conversion by the conversion circuit and an auralizing instrument for converting compressive force and pulling force resulting from conversion by the conversion circuit into voice and sound.

Preferably, the linear body is a linear medical appliance for insertion into a vessel in a body.

In addition, a measurement device according to another aspect of this invention is a measurement device for measuring compressive force and pulling force in a direction of a longitudinal axis applied to a linear body having flexibility, it includes a main body in which a through hole for inserting the linear body is formed, the through hole being formed to allow bending of the linear body in an arc shape in the through hole and variation in degree of bending of the linear body in accordance with the compressive force and the pulling force, a sensor for detecting a degree of bending of the linear body, and a conversion circuit for converting the degree of bending detected by the sensor into a signal indicating compressive force and pulling force applied to the linear body, and the through hole is formed such that the degree of bending of the linear body increases when compressive force is applied to the linear body as compared with when compressive force and pulling force are not applied to the linear body and such that the degree of bending of the linear body decreases when the pulling force is applied to the linear body as compared with when compressive force and pulling force are not applied to the linear body.

In order to solve the problems above, a medical device according to one aspect of this invention includes the measurement device above.

In order to solve the problems above, a training device according to one aspect of this invention includes the measurement device above.

In order to solve the problems above, a measurement method according to one aspect of this invention is a measurement method of measuring compressive force and pulling force in a direction of a longitudinal axis applied to a linear body having flexibility, and the measurement method includes the steps of inserting the linear body into a through hole formed to allow bending of the linear body in an arc shape therein and variation in degree of bending of the linear body in accordance with the compressive force and the pulling force, detecting a degree of bending of the linear body, and converting the detected degree of bending into a signal indicating compressive force and pulling force applied to the linear body based on prescribed correlation between the degree of bending of the linear body in the through hole and compressive force and pulling force.

### ADVANTAGEOUS EFFECTS OF INVENTION

According to the present invention, a linear body can satisfactorily be operated with a simplified construction.

### BRIEF DESCRIPTION OF DRAWINGS

Fig. 1 is an external view showing a construction of a main body of a measurement device according to a first embodiment of the present invention.
Fig. 2 is a cross-sectional view showing a cross-section along the line II-II in Fig. 1.
Fig. 3 is a cross-sectional view showing a cross-section along the line III-III in Fig. 1.
Fig. 4 is a schematic diagram showing an overall configuration of the measurement device.
Fig. 5 is a diagram showing such a state that compressive force is applied to a linear body 1 and linear body 1 is bent in a main body 2 in the cross-sectional view in Fig. 2.
Fig. 6 is a diagram showing such a state that pulling force is applied to linear body 1 and linear body 1 is bent in main body 2 in the cross-sectional view in Fig. 2.
Fig. 7 is a diagram showing correlation between force applied to the linear body and a position of the linear body detected by a line sensor.
Fig. 8 is a diagram showing a procedure of a measurement method according to the first embodiment of the present invention.
Fig. 9 is a diagram showing a construction of a coil for embolization of a cerebral aneurysm with a coil.
Fig. 10 is a cross-sectional view showing a construction of a measurement device according to a second embodiment of the present invention.

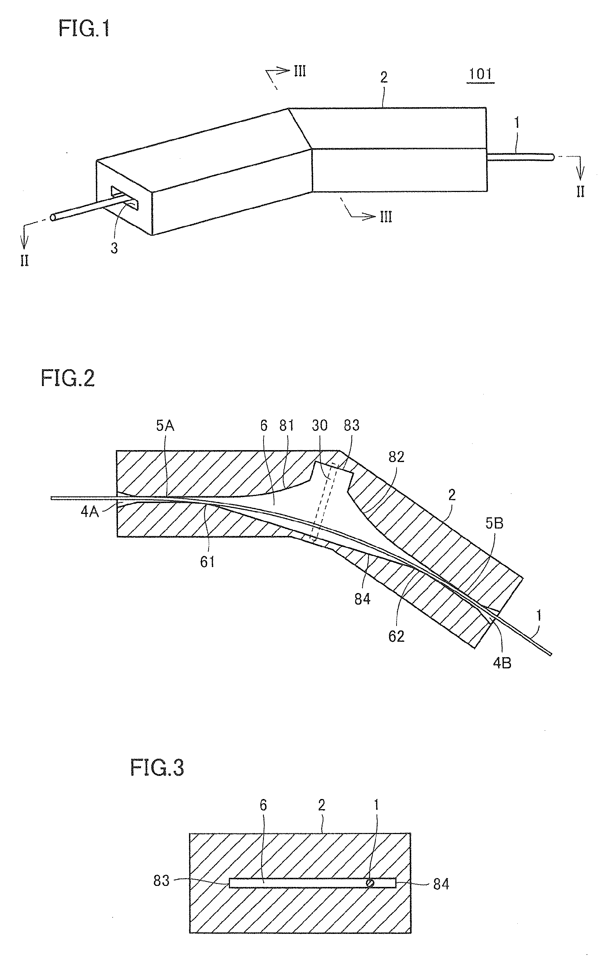

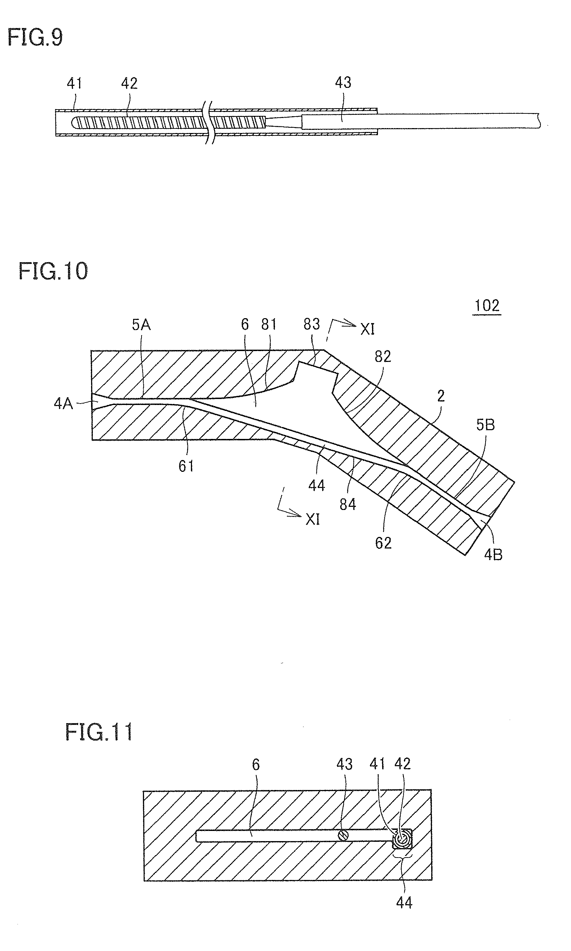

Fig. 1 1 is a cross-sectional view showing a cross-section along the line XI-XI in Fig. 10.

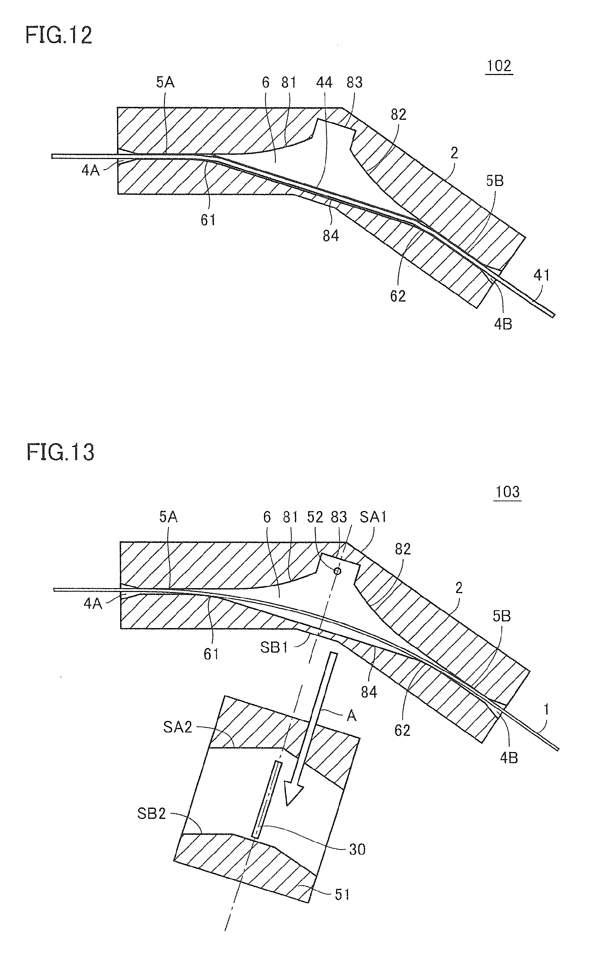

Fig. 12 is a diagram showing such a state shat a sheath 41 is inserted into main body 2 in the cross-sectional view in Fig. 10.
Fig. 13 is a cross-sectional view showing a construction of a measurement device according to a third embodiment of the present invention.

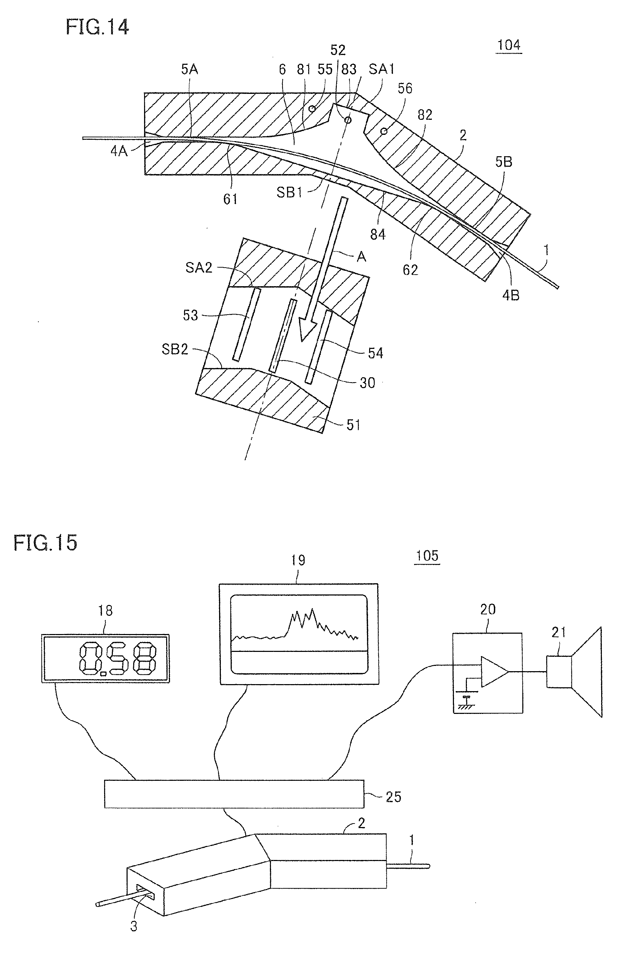

Fig. 14 is a cross-sectional view showing a construction of a measurement device according to a fourth embodiment of the present invention.
Fig. 15 is a diagram showing an example where an operator is notified of a result of measurement by the measurement device.

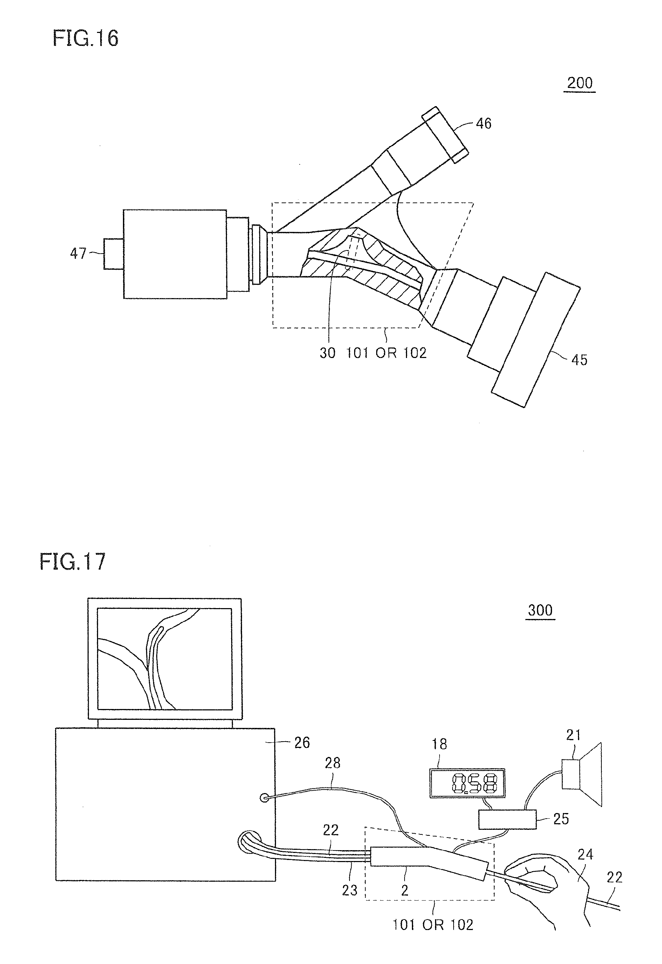

Fig. 16 is a diagram showing a construction of a Y-connector according to a sixth embodiment of the present invention.
Fig. 17 is a diagram showing a construction of a training device according to a seventh embodiment of the present invention.

### DESCRIPTION OF EMBODIMENTS

An embodiment of the present invention will be described hereinafter with reference to the drawings. In the drawings, the same or corresponding elements have the same reference characters allotted and description thereof will not be repeated.

### <First Embodiment>

Fig. 1 is an external view showing a construction of a main body of a measurement device according to a first embodiment of the present invention.

Referring to Fig. 1, a measurement device 101 includes a main body 2, in which a through hole 3 into which a linear body 1 having flexibility is inserted is formed. Fig. 1 shows a state that measurement device 101 is set on a floor surface. Though not shown, a line sensor 30 which will be described later is arranged on a floor surface side of measurement device 101, and a light source 29 which will be described later is arranged on a ceiling surface side of measurement device 101. Main body 2 is, for example, a transparent body, and it is formed of a substance through which light can pass.

Fig. 2 is a cross-sectional view showing a cross-section along the line II-II in Fig. 1. Fig. 3 is a cross-sectional view showing a cross-section along the line III-III in Fig. 1.

Referring to Fig. 2, through hole 3 has tapered input/output ports 4A, 4B as a first end portion and a second end portion respectively, in order to improve insertion performance by making an entrance and an exit into which linear body 1 is inserted greater. Through hole 3 has restraint portions 5A, 5B provided between input/output ports 4A, 4B, for restricting movement of linear body 1 in a direction other than a direction of a longitudinal axis of linear body 1. In restraint portions 5A, 5B, through hole 3 is slightly greater in diameter than linear body 1 (for example, 105% to 120% of a diameter of linear body 1). In addition, a length of through hole 3 along the direction of the longitudinal axis of linear body 1 is at least several times as great as the diameter of linear body 1. Therefore, in restraint portions 5A, 5B, an operation of linear body 1 is restrained in a direction other than the direction of the longitudinal axis.

Through hole 3 is formed to allow bending of linear body 1 in an arc shape in through hole 3 and variation in degree of bending of linear body 1 in accordance with compressive force and pulling force applied to linear body 1. Namely, through hole 3 is formed such that linear body 1 is bent in an arc shape in a detection portion 6 within through hole 3 while compressive force and pulling force in the direction of the longitudinal axis are not applied to linear body 1. Further, through hole 3 is formed such that the degree of bending of linear body 1 increases when compressive force is applied to linear body 1 as compared with when compressive force and pulling force are not applied to linear body 1 and the degree of bending of linear body 1 decreases when pulling force is applied to linear body 1 as compared with when compressive force and pulling force are not applied to linear body 1.

According to such a construction, even when compressive force and pulling force in the direction of the longitudinal axis applied to linear body 1 are very small, compressive force and pulling force can accurately be detected.

Main body 2 defines a direction of bending of linear body 1 within through hole 3 when compressive force and pulling force in the direction of the longitudinal axis are applied to linear body 1. Namely, through hole 3 is bent between two restraint portions 5A, 5B, and as linear body 1 is inserted into through hole 3, linear body 1 is in a bent shape. In addition, through hole 3 is formed to make up such a detection portion 6 that inner walls 81, 82 are distant from an inner wall 84 and a diameter of through hole 3 increases between two restraint portions 5A, 5B therein.

Restraint portions 5A, 5B are provided in input/output port 4A and input/output port 4B respectively, in a manner adjacent thereto, and each has an opening area S1 when it is cut in a plane orthogonal to a direction of insertion of linear body 1. Detection portion 6 is provided between restraint portion 5A and restraint portion 5B and it has an opening area S2 greater than opening area S1 when it is cut in a plane orthogonal to the direction of insertion of linear body 1. Then, detection portion 6 is formed such that linear body 1 is bent in an arc shape, and expanded in a direction of an inner periphery and a direction of an outer periphery of linear body 1 bent in the arc shape.

Detection portion 6 is formed as inner walls 81, 82 located on an outer side of bending of linear body 1 extend. Inner wall 84 is formed as a plane. In detection portion 6, an operation of linear body 1 in a direction parallel to the sheet surface of Fig. 2 is not restricted. Linear body 1 passes through the inside of main body 2 while it is bent in detection portion 6.

In input/output ports 4A, 4B and detection portion 6, a height of through hole 3 in a direction perpendicular to the sheet surface of Fig. 2 is slightly greater than the diameter of linear body 1 (for example, 105% to 120% of the diameter of linear body 1), and an operation of linear body 1 in a direction perpendicular to the sheet surface of Fig. 2 is restrained. Namely, in input/output ports 4A, 4B and detection portion 6, a cross-sectional shape of through hole 3 in a cross-section perpendicular to the direction of the longitudinal axis of linear body 1 is rectangular. Thus, a direction of bending of linear body 1 within through hole 3 is defined, and linear body 1 is positioned such that a height of a crest of a bending portion of linear body 1 when compressive force and pulling force in the direction of the longitudinal axis are applied to linear body 1 (that is, a maximum value of a distance from inner wall 84 to linear body 1) is determined.

Line sensor 30 is arranged in detection portion 6 so as to extend across the cross-section of through hole 3 in detection portion 6. Line sensor 30 is arranged such that it extends across the inside of detection portion 6 from inner wall 84 of through hole 3 to the inside of a recess portion 83 making up an inner wall of through hole 3 opposed to inner wall 84, which will be described later. Line sensor 30 is arranged along a trace of peaks of the crest of bending that are formed as linear body 1 is bent in the arc shape when compressive force and pulling force in the direction of the longitudinal axis are applied to linear body 1 in detection portion 6.

In addition, as shown in Fig. 2, boundary portions 61, 62 between inner wall 84 in detection portion 6 and restraint portions 5A, 5B are formed in a curved surface shape convex toward the inside of through hole 3. Inner walls 81, 82 are formed in a curved surface shape convex toward the inside of through hole 3. Inner wall 81 is formed in a curved surface shape as being in contact with the inner wall of through hole 3 in restraint portion 5A, while inner wall 82 is formed in a curved surface shape as being in contact with the inner wall of through hole 3 in restraint portion 5B. Thus, bending of linear body 1 accompanying plastic deformation can be prevented. In addition, recess portion 83 is formed between inner wall 81 and inner wall 82 in detection portion 6. Recess portion 83 is formed as the inner wall of through hole 3 is recessed toward an external side of main body 2 such that the inner wall of through hole 3 between inner wall 81 and inner wall 82 in detection portion 6 is more distant from inner wall 84.

The wall portion of detection portion 6 is shaped such that inner walls 81, 82 each in a curved surface shape convex toward the inside of through hole 3 and recess portion 83 are combined. Owing to such a shape of detection portion 6, when linear body 1 is bent as compressive force in the direction of the longitudinal axis is applied to linear body 1 in detection portion 6, linear body 1 can be bent along the inner wall (that is, inner wall 81 and inner wall 82) of through hole 3 located outside bending of linear body 1. Further, a part of linear body 1 can be bent away from inner wall 81 and inner wall 82. Furthermore, as compressive force increases, a distance between contact points, that are points where linear body 1 is away from inner walls 81,82 decreases.

Therefore, buckling of linear body 1 in detection portion 6 can be suppressed. Namely, even in a case where linear body 1 small in buckling load is employed, linear body 1 can be bent in detection portion 6 without buckling and hence a degree of bending of linear body 1 can accurately be detected. By converting the detected degree of bending, compressive force in the direction of the longitudinal axis applied to linear body 1 can be measured.

In addition, since recess portion 83 is formed in detection portion 6, compressive force applied to linear body 1 can accurately be measured over a wide range. Namely, by detecting a height of a crest of bending of linear body 1 in detection portion 6, compressive force applied to linear body 1 is measured. Here, unless a peak of the bending portion of linear body 1 located within detection portion 6, that is, a point on linear body 1 located within detection portion 6 most distant from inner wall 84, is in contact with the inner wall of detection portion 6, compressive force applied to linear body 1 can be measured. If recess portion 83 is formed, greater compressive force in the direction of the longitudinal axis will be required in order to bring the peak of the bending portion of linear body 1 in contact with the inner wall of detection portion 6. Therefore, a range of measurement of compressive force applied to linear body 1 can be widened.

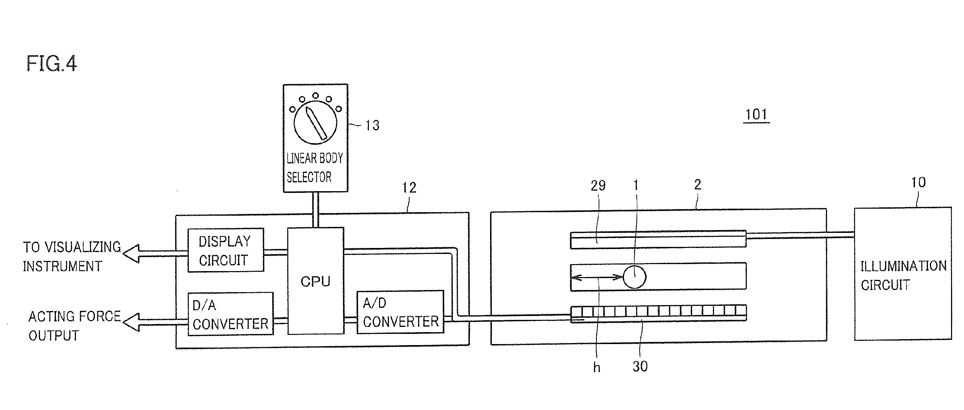

Fig. 4 is a schematic diagram showing an overall configuration of the measurement device.

Referring to Fig. 4, measurement device 101 further includes light source 29 emitting light, line sensor 30 serving as a light receiver for receiving light emitted from light source 29, an illumination circuit 10 causing light source 29 to emit light, and a conversion circuit 12.

Line sensor 30 is a one-dimensional optical array sensor having a plurality of light-receiving elements receiving light arranged in line.

An optical path extending from light source 29 to line sensor 30 in the inside of main body 2 is composed of a light-transmissive material through which light used for detection passes.

Light source 29 and line sensor 30 are arranged with detection portion 6 lying therebetween, so as to oppose to each other with linear body 1 lying therebetween.

Line sensor 30 is arranged along a direction of height of the crest of bending of linear body 1, that is, a direction of movement of the peak of the crest of bending of linear body 1 when compressive force and pulling force in the direction of the longitudinal axis are applied to linear body 1. Line sensor 30 is arranged in a direction perpendicular to a direction of extension of inner wall 84 and arranged to be orthogonal to linear body 1 at the peak of the crest of bending. Line sensor 30 detects a degree of bending of linear body 1 by measuring a height h of the crest of bending of linear body 1.

A degree of bending of linear body 1 is detected based on shadow of linear body 1 projected on line sensor 30. Namely, when line sensor 30 receives light emitted by light source 29 and when linear body 1 is located over a certain light-receiving element of line sensor 30, the light emitted by light source 29 is cut off by linear body 1. Then, a quantity of light received by that light-receiving element decreases. A position of that light-receiving element corresponds to the degree of bending of linear body 1.

The construction is not limited to such a construction that the light receiver arranged at a position opposed to the light source receives transmitting light, and the light source and the light receiver may be arranged side by side and such a reflector as a mirror for reflecting light emitted by the light source may be set at a position opposed to the light source. In this case, as the light receiver receives reflection light reflected by the reflector, of the light emitted by the light source, a degree of bending of the linear body can similarly be detected. Alternatively, a degree of bending of the linear body can also be detected with a two-dimensional array sensor in which a plurality of light-receiving elements are arranged, for example, in matrix on a plane, instead of the one-dimensional array sensor such as a line sensor. In addition, since a degree of bending of the linear body should only be detected, for example, a non-contact distance sensor for detecting a height of a crest of bending, a position sensor for detecting a position of the linear body, or the like can also be employed.

Illumination circuit 10 and conversion circuit 12 are provided outside main body 2. Illumination circuit 10 causes light source 29 to emit light. Conversion circuit 12 converts a degree of bending of linear body 1 detected based on a quantity of light received by line sensor 30 with respect to the quantity of light emitted by light source 29 into a signal indicating compressive force and pulling force in the direction of the longitudinal axis applied to linear body 1 and outputs the signal. It is noted that conversion circuit 12 may have an amplifier circuit for amplifying an output from line sensor 30.

Conversion circuit 12 converts a degree of bending of linear body 1 into a signal indicating compressive force and pulling force applied to linear body 1 based on prescribed correlation between the degree of bending of linear body 1 and compressive force and pulling force applied to linear body 1. It is noted that, in order to have an image of linear body 1 appropriately formed on line sensor 30, such an optical element as a lens, a slit, and a filter for cutting off external light may be set in the present optical system.

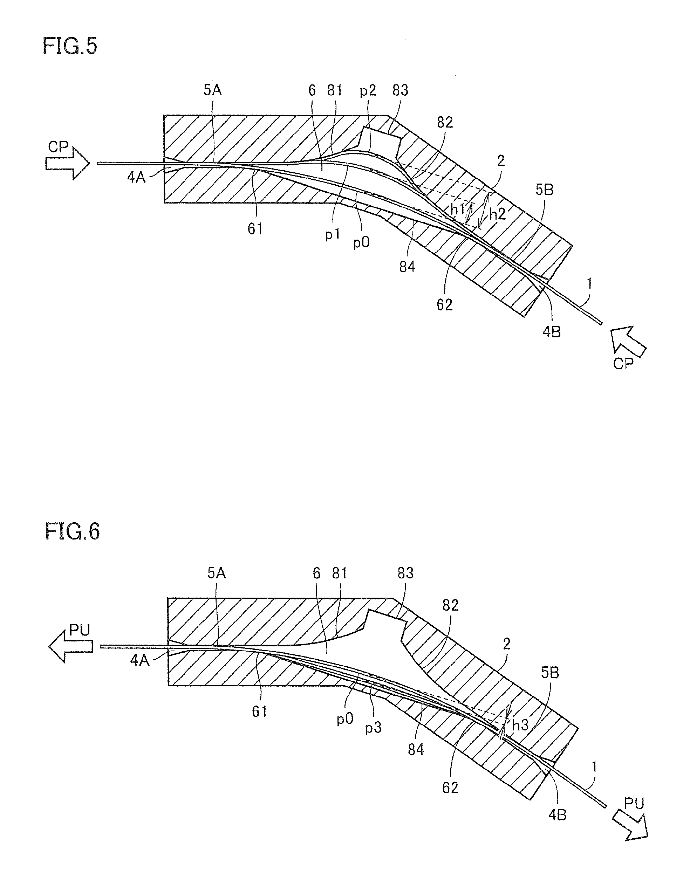

Fig. 5 is a diagram showing such a state that compressive force is applied to linear body 1 and linear body 1 is bent in main body 2 in the cross-sectional view in Fig. 2.

Referring to Fig. 5, when compressive force CP in the direction of the longitudinal axis is applied to linear body 1, a degree of bending of linear body 1 increases. With increase in degree of bending of linear body 1, a height of the crest of bending becomes greater.

In Fig. 5, a state of linear body 1 while compressive force and pulling force are not applied to linear body 1 is indicated with p0 In state p0, linear body 1 is bent in an arc shape.

When compressive force CP is applied to linear body 1, linear body 1 is further bent relative to state p0and the height of the crest of bending increases by h1 as compared with state p0 (a state p1).

When compressive force CP greater than in state p1is applied to linear body 1, linear body 1 is further bent relative to state p1,the height of the crest of bending further increases as compared with state p1, and the height increases by h2 (h2 > h1)as compared with state p0 (a state p2).

Fig. 6 is a diagram showing such a state that pulling force is applied to linear body 1 and linear body 1 is bent in main body 2 in the cross-sectional view in Fig. 2.

Referring to Fig. 6, when pulling force PU in the direction of the longitudinal axis is applied to linear body 1, a degree of bending of linear body 1 decreases. With decrease in degree of bending of linear body 1, the height of the crest of bending becomes smaller.

In Fig. 6, a state of linear body 1 while compressive force and pulling force are not applied to linear body 1 is indicated with p0. In state p0, linear body 1 is bent in an arc shape.

When pulling force PU is applied to linear body 1, a degree of bending of linear body 1 decreases relative to state p0 and the height of the crest of bending decreases by h3 as compared with state p0 (a state p3).

When the degree of bending of linear body 1 increases as compared with the degree of bending of linear body 1 while compressive force and pulling force are not applied to linear body 1, conversion circuit 12 converts the detected degree of bending into a signal indicating compressive force applied to linear body 1. Alternatively, when the degree of bending of linear body 1 decreases as compared with the degree of bending of linear body 1 while compressive force and pulling force are not applied to linear body 1, conversion circuit 12 converts the detected degree of bending into a signal indicating pulling force applied to linear body 1.

For example, line sensor 30 detects a position of the bending portion of linear body 1 within through hole 3. When linear body 1 is displaced from a reference position of linear body 1 while compressive force and pulling force are not applied to linear body 1 toward an outer periphery, conversion circuit 12 converts an amount of displacement from the reference position into a signal indicating compressive force applied to linear body 1, and when linear body 1 is displaced from the reference position toward an inner periphery, conversion circuit 12 converts an amount of displacement from the reference position into a signal indicating pulling force applied to linear body 1.

When line sensor 30 receives light emitted by light source 29 arranged at a position opposed to line sensor 30 with detection portion 6 lying therebetween and when linear body 1 is located over a certain light-receiving element of the plurality of light-receiving elements in line sensor 30, the light emitted by light source 29 is cut off by linear body 1. Then, a quantity of light received by that light-receiving element decreases. By detecting a position of that light-receiving element, a position of linear body 1 can be specified and increase and decrease in height of the crest of bending of linear body 1, that is, a degree of bending of linear body 1, can be detected.

Conversion circuit 12 converts a height of the crest of bending of linear body 1 into a signal indicating compressive force and pulling force applied to linear body 1 based on prescribed correlation between the height of the crest of bending of linear body 1 detected by line sensor 30 and compressive force and pulling force applied to linear body 1 (for example, a set of compressive force of certain magnitude and a height of the crest of bending corresponding thereto) and outputs the signal. Compressive force and pulling force applied in the direction of the longitudinal axis of linear body 1 can thus be measured.

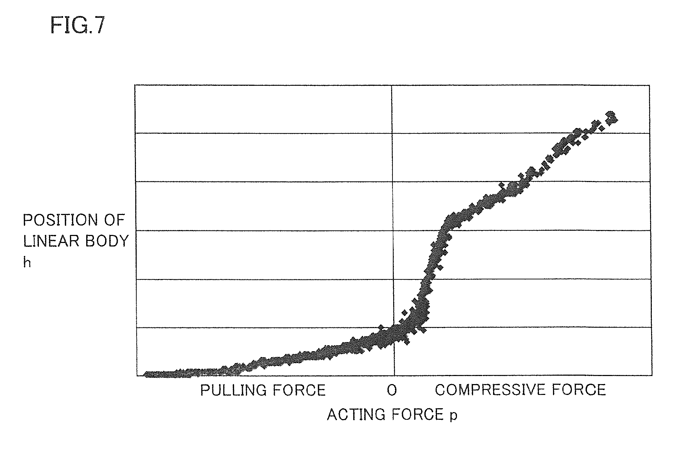

Fig. 7 is a diagram showing correlation between force applied to the linear body and a position of the linear body detected by the line sensor.

Fig. 7 shows results of measurement of force applied to the linear body and a position of the linear body detected by the line sensor. In Fig. 7, the abscissa represents acting force p in the direction of the longitudinal axis applied to linear body 1, and the ordinate represents height h of the crest of bending of linear body 1.

Conversion circuit 12 stores correlation shown in Fig. 7 and converts an electric signal received from line sensor 30 into a signal indicating compressive force and pulling force applied to linear body 1 based on this correlation. It is noted that conversion circuit 12 may store correlation shown in Fig. 7 as it is or store an equation approximating correlation shown in Fig. 7.

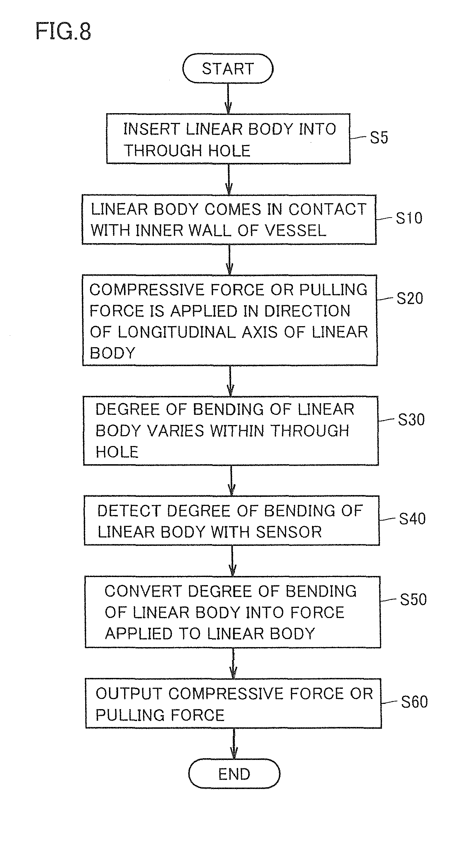

Fig. 8 is a diagram showing a procedure of a measurement method according to the first embodiment of the present invention.

Referring to Fig. 8, initially, in the step (S5), linear body 1 is inserted into through hole 3 of main body 2. Namely, linear body 1 is inserted into through hole 3 formed to allow bending of linear body 1 in an arc shape therein and variation in degree of bending of linear body 1 in accordance with compressive force and pulling force.

Then, in the step (S10), a tip end of linear body 1 comes in contact with an inner wall of a vessel as a result of insertion of linear body 1 in the vessel and operation of the linear body 1 from the outside of the vessel.

Then, in the step (S20), when force is applied in the direction of the longitudinal axis of linear body 1 from the outside of the vessel in order to further insert or pull out linear body 1, an operation of linear body 1 is restricted depending on whether the tip end of linear body 1 is in contact with the inner wall of the vessel, linear body 1 is caught in the vessel in the body, or the like. Therefore, compressive force or pulling force is applied in the direction of the longitudinal axis of linear body 1.

Then, in the step (S30), as a result of acting compressive force or pulling force, a degree of bending of linear body 1 varies in detection portion 6 within through hole 3.

Then, in the step (S40), line sensor 30 detects the degree of bending of linear body 1.

Then, in the step (S50), conversion circuit 12 converts the degree of bending of linear body 1 detected in the step (S40) into compressive force or pulling force applied to linear body 1 based on prescribed correlation between the degree of bending of linear body 1 and compressive force and pulling force applied to linear body 1.

Then, in the step (S60), a signal indicating compressive force or pulling force, which results from conversion from the degree of bending of linear body 1, is output.

As above, with the measurement device and the measurement method according to the first embodiment of the present invention, when compressive force or pulling force in the direction of the longitudinal axis is applied to linear body 1, line sensor 30 detects a degree of bending of linear body 1. Then, conversion circuit 12 converts the detected degree of bending of linear body 1 into a signal indicating compressive force or pulling force in the direction of the longitudinal axis applied to linear body 1. In addition, since line sensor 30 is provided at a position where linear body 1 is operated, which is located outside the vessel in which linear body 1 is inserted, so as to measure compressive force and pulling force in the direction of the longitudinal axis applied to linear body 1, compressive force and pulling force can quantitatively be measured also for extremely thin linear body 1 in which it is difficult to provide a pressure sensor at the tip end.

Namely, with the measurement device and the measurement method according to the first embodiment of the present invention, force for inserting the linear body and force for pulling out the linear body can both be measured. In addition, since the construction is simple, incorporation into medical equipment is easy and hence a warning can be issued when excessive load is applied to a human body and a linear body serving as a treatment device in an operation for inserting and pulling out the linear body to be inserted in a human body. Therefore, with the measurement device and the measurement method according to the first embodiment of the present invention, the linear body can satisfactorily be operated with a simplified construction.

If a shape and a material, that is, a Young's modulus, of linear body 1 are different, a degree of bending of linear body 1 at the time when the same force is applied will be different. Therefore, when a plurality of linear bodies 1 different in shape and material are used, correlation between degrees of bending of various linear bodies different in shape and material and force in the direction of the longitudinal axis applied to the linear bodies is determined in advance and stored in conversion circuit 12. Then, measurement device 101 includes also a linear body selector 13 shown in Fig. 4, so that linear body selector 13 selects which correlation is to be used, in accordance with linear body 1 that is used. Thus, as the same measurement device is applicable to linear bodies 1 of various shapes and materials, linear bodies 1 that have been used for various different applications so far can be used without modification, and cost effectiveness is achieved.

Another embodiment of the present invention will now be described with reference to the drawings. It is noted that the same or corresponding elements in the drawings have the same reference characters allotted and description thereof will not be repeated.

### <Second Embodiment>

The present embodiment relates to a measurement device different in shape of a through hole from the measurement device according to the first embodiment. The measurement device is similar to the measurement device according to the first embodiment except for contents which will be described below.

The measurement device according to a second embodiment of the present invention is suitable for embolization of a cerebral aneurysm with a coil, which is a treatment method for preventing rupture of a cerebral aneurysm representing a cause of subarachnoid hemorrhage. The present treatment is treatment for densely filling an aneurysm with a coil with a catheter serving as a guide tube.

Fig. 9 is a diagram showing a construction of a coil for embolization of a cerebral aneurysm with a coil.

Referring to Fig. 9, a coil for embolization of a cerebral aneurysm with a coil includes a coil portion 42 used for embolization and a delivery wire portion 43 for feeding coil portion 42.

A coil for embolization of a cerebral aneurysm with a coil has a spiral shape or a cage shape in conformity with a size of an aneurysm, and before use, it extends straight as shown in Fig. 9, for easy insertion in a catheter.

An operator operates delivery wire portion 43 so as to fill the aneurysm with coil portion 42. After the operator inserts coil portion 42, he/she changes the coil portion with a coil portion different in size, and after the operator leaves coil portion 42, he/she pulls out delivery wire portion 43. In particular, since coil portion 42 is flexible, forced pull-out leads to extension of the coil portion. Therefore, it is important to perform an operation for pulling out coil portion 42 without applying excessive pulling force to coil portion 42.

In addition, in coil embolization, an aneurysm susceptible to rupture is densely filled with coil portion 42. Therefore, in order not to apply excessive force to the aneurysm, it is also important to measure insertion force in inserting delivery wire portion 43, that is, compressive force applied to the linear body.

Here, since coil portion 42 is flexible, it is accommodated in a sheath 41 before use. In a case where coil portion 42 is inserted in a human body through a catheter, sheath 41 and an inlet of the catheter are aligned with each other such that coil portion 42 is not out of place. Then, delivery wire portion 43 is pushed forward to move coil portion 42 into the catheter. Then, when coil portion 42 completely entered the catheter, sheath 41 is removed from delivery wire portion 43 so that delivery wire portion 43 is operated.

Since sheath 41 is greater in diameter than delivery wire portion 43, sheath 41 cannot be inserted in main body 2 of measurement device 101 according to the first embodiment of the present invention. If sheath 41 is removed and coil portion 42 is inserted in measurement device 101, coil portion 42 will spread in detection portion 6.

Then, in the measurement device according to the second embodiment of the present invention, a through hole is formed as follows.

Fig. 10 is a cross-sectional view showing a construction of the measurement device according to the second embodiment of the present invention. Fig. 10 corresponds to Fig. 2 in the first embodiment of the present invention. Fig. 11 is a cross-sectional view showing a cross-section along the line XI-XI in Fig. 10. Fig. 11 corresponds to Fig. 3 in the first embodiment of the present invention. Fig. 12 is a diagram showing such a state shat sheath 41 is inserted into main body 2 in the cross-sectional view in Fig. 10.

Referring to Fig. 10, through hole 3 in a measurement device 102 includes a sheath groove 44 which is a groove dedicated for passage of sheath 41. More specifically, sheath groove 44 is provided between restraint portion 5A and restraint portion 5B and connected to detection portion 6, and a length thereof in a direction at a right angle to a direction of insertion of linear body 1 and a direction of radius of linear body 1 bent in an arc shape is greater than detection portion 6.

Therefore, as shown in Fig. 11, sheath 41 can pass through only sheath groove 44 between restraint portions 5A and sub. Then, passage of sheath 41 through main body 2 is as shown in Fig. 12.

Measurement device 102 and a catheter are connected to each other, and sheath 41 and a mouth of the catheter are aligned with each other. Then, after delivery wire portion 43 is pushed to cause coil portion 42 to completely enter the catheter, sheath 41 is pulled out of main body 2. Then, delivery wire portion 43 remains in main body 2. Here, delivery wire portion 43 bends owing to its rigidity. Since delivery wire portion 43 is thinner than sheath 41, it moves from sheath groove 44 to detection portion 6 and it is bent as in measurement device 101 as shown in Fig. 2.

A portion of through hole 3 other than sheath groove 44 has a width slightly greater than a diameter of delivery wire portion 43. Therefore, as in measurement device 101, a position of a linear body in detection portion 6 is uniquely determined in correspondence with acting force, and sheath 41 can pass through without deteriorating accuracy in detection of acting force.

In addition, measurement device 102 is provided with sheath groove 44 in a portion of detection portion 6 where delivery wire portion 43 does not pass through. Namely, sheath groove 44 is provided opposite to the direction of bending of linear body 1. Sheath groove 44 is connected to the end portion of detection portion 6 on the inner periphery side of linear body 1 bent in an arc shape. According to such a construction, sheath groove 44 can readily be made.

As the construction and the operation are otherwise the same as those of the measurement device according to the first embodiment, detailed description will not be repeated here.

Another embodiment of the present invention will now be described with reference to the drawings. It is noted that the same or corresponding elements in the drawings have the same reference characters allotted and description thereof will not be repeated.

### <Third Embodiment>

The present embodiment relates to a measurement device different in an attachable/removable sensor from the measurement device according to the first embodiment. The measurement device is similar to the measurement device according to the first embodiment except for contents which will be described below.

Fig. 13 is a cross-sectional view showing a construction of the measurement device according to a third embodiment of the present invention. Fig. 13 corresponds to Fig. 2 in the first embodiment of the present invention.

Referring to Fig. 13, a measurement device 103 further includes a housing 51 as compared with the measurement device according to the first embodiment of the present invention. Line sensor 30 is contained, for example, in housing 51. Namely, housing 51 is provided with line sensor 30 which is attachable to/removable from main body 2.

In using measurement device 103, surfaces SA1 and SB1 of main body 2 and surfaces SA2 and SB2 of housing 51 are aligned and integrated, respectively.

By thus separating main body 2 and line sensor 30 from each other, main body 2 alone is disposable. Therefore, in a case where the measurement device is applied for medical use, sanity of main body 2 through which linear body 1 inserted in a human body passes can be maintained inexpensively.

In order to calibrate misalignment between line sensor 30 and main body 2 at the time when line sensor 30 is attached to main body 2, a marker 52 is provided on a main body 2 side. While line sensor 30 is attached to main body 2, line sensor 30 detects a position of marker 52 and corrects the detected degree of bending of linear body 1 based on the detected position of marker 52. Namely, marker 52 is placed at a position readable by line sensor 30. Misalignment above can be corrected based on a read image of marker 52.

Though not shown, the construction may be such that a reflector is set at a position of light source 29 shown in Fig. 4, light source 29 is set in housing 51 as aligned with line sensor 30, light output from light source 29 is reflected by the reflector, and line sensor 30 receives this reflected light. In this case, since light source 29 and line sensor 30 that are photoelectronic components can be set in housing 51, the reflector can be fixed to main body 2 and hence separation between line sensor 30 and main body 2 is facilitated.

As the construction and the operation are otherwise the same as those of the measurement device according to the first embodiment, detailed description will not be repeated here.

Another embodiment of the present invention will now be described with reference to the drawings. It is noted that the same or corresponding elements in the drawings have the same reference characters allotted and description thereof will not be repeated.

### <Fourth Embodiment>

The present embodiment relates to a measurement device different in a plurality of sensors from the measurement device according to the third embodiment. The measurement device is similar to the measurement device according to the first embodiment except for contents which will be described below.

Fig. 14 is a cross-sectional view showing a construction of the measurement device according to a fourth embodiment of the present invention. Fig. 14 corresponds to Fig. 2 in the first embodiment of the present invention.

Referring to Fig. 14, a measurement device 104 further includes line sensors 53 and 54 as compared with the measurement device according to the third embodiment of the present invention.

Line sensors 30, 53, 54 are contained, for example, in housing 51. Namely, housing 51 is provided with line sensors 30, 53, 54, which are attachable to/removable from main body 2.

In order to calibrate misalignment between line sensors 30, 53, 54 and main body 2 when line sensors 30, 53, 54 are attached to main body 2, markers 52, 55, 56 are provided on the main body 2 side. While line sensors 30, 53, 54 are attached to main body 2, line sensors 30, 53, 54 detect respective positions of markers 52, 55, 56 and correct the detected degree of bending of linear body 1 based on the detected positions of markers 52, 55, 56. Namely, markers 52, 55, 56 are placed at positions readable by line sensors 30, 53, 54, respectively. Misalignment above can be corrected based on read images of markers 52, 55., 56.

Conversion circuit 12 converts a degree of bending detected by line sensors 30, 53, 54 into compressive force and pulling force applied to linear body 1. By mounting a plurality of line sensors or a two-dimensional sensor on a sensor housing and arranging a plurality of markers in correspondence with the sensor(s), misalignment between line sensors 30, 53, 54 and main body 2 can highly accurately be corrected when line sensors 30, 53, 54 are attached to main body 2.

In addition, by using a plurality of line sensors or a two-dimensional sensor, compressive force and pulling force applied to linear body 1 can highly accurately be measured.

As the construction and the operation are otherwise the same as those of the measurement device according to the third embodiment, detailed description will not be repeated here.

Another embodiment of the present invention will now be described with reference to the drawings. It is noted that the same or corresponding elements in the drawings have the same reference characters allotted and description thereof will not be repeated.

### <Fifth Embodiment>

In the present embodiment, an example in which an operator is notified of a result of measurement by the measurement device will be shown. The measurement device is similar to the measurement device according to the first embodiment except for contents which will be described below.

Fig. 15 is a diagram showing an example where an operator is notified of a result of measurement by the measurement device.

Referring to Fig. 15, a measurement device 105 includes a visualizing instrument (a display portion) 18 and a visualizing instrument (a display portion) 19. Visualizing instrument 18 displays compressive force and pulling force resulting from conversion by conversion circuit 12. For example, visualizing instrument 18 displays a numeric value obtained by converting a voltage output from line sensor 30 into compressive force and pulling force applied to linear body 1. Visualizing instrument 19 converts compressive force and pulling force resulting from conversion by conversion circuit 12 into a video image. For example, visualizing instrument 19 displays in a graph compressive force and pulling force with time history.

In addition, measurement device 105 includes an auralizing instrument 20 for varying an acoustic effect, that is, sounding a warning alarm from a speaker 21 when compressive force or pulling force obtained by converting a voltage output from line sensor 30 exceeds a prescribed threshold value, that is, when compressive force and pulling force applied to linear body 1 each exceed a prescribed threshold value. For example, in a case where acting force is close to zero N (newton), no sound is generated. As compressive force increases, an acoustic frequency is raised. On the other hand, as pulling force increases, an acoustic frequency is lowered. In particular in a case where an operator pays attention to a fluoroscopic image as in coil embolization or the like, it is convenient to know insertion force through the acoustic effect.

In addition, measurement device 105 includes a converter 25 for converting a signal from conversion circuit 12 in accordance with equipment to be connected and outputting a resultant signal to visualizing instrument 18, visualizing instrument 19, and auralizing instrument 20.

It is noted that compressive force and pulling force applied to linear body 1 each exceeding a prescribed threshold value can be indicated also by change in visual effect such as turn-on of an indicator. Further, a speaker and an indicator may be employed together. Furthermore, a plurality of threshold values can be determined in advance and sound from a speaker and a color of an indicator can be changed in correspondence with each threshold value.

Abrupt change in visual effect or acoustic effect around a threshold value can reliably call attention of an operator, which is effective. For example, such measures as change in color of light emitted from an indicator or change in tone of a warning alarm (intermittent sound and continuous sound, low-pitch sound and high-pitch sound, etc.) around a threshold value are possible.

Though main body 2 is connected through a cable to visualizing instrument 18, visualizing instrument 19, and auralizing instrument 20 in Fig. 15, other signal transmission means such as wireless communication through infrared or other electromagnetic waves can also be employed.

Thus, kinesthetic sense information of insertion resistance of linear body 1 externally held with fingers of the operator can be quantified and displayed. In addition, the kinesthetic sense information can be recorded as data, for example as a matter printed on paper in a graph or a numeric value or as electronic data in a hard disk or a memory. Therefore, manipulation of a skilled operator can quantitatively be transferred to a less experienced operator.

In addition, when compressive force applied to linear body 1 exceeds a predetermined threshold value, the operator can reliably be notified of that fact through a warning alarm and turn-on of an indicator. Therefore, excessive load can be prevented from acting on a vessel.

Another embodiment of the present invention will now be described with reference to the drawings. It is noted that the same or corresponding elements in the drawings have the same reference characters allotted and description thereof will not be repeated.

### <Sixth Embodiment>

An example in which a measurement device for measuring compressive force and pulling force in the direction of the longitudinal axis applied to the linear body serving as a linear medical appliance to be inserted in a vessel in a body is used as being incorporated in other medical equipment is shown as an example where the measurement device according to the present invention is put into practical use.

Fig. 16 is a diagram showing a construction of a Y-connector according to a sixth embodiment of the present invention.

Referring to Fig. 16, a Y-connector (a medical device) 200 includes measurement device 101 or measurement device 102, input ports 45 and 46, and an output port 47.

The Y-connector serves for inserting a linear body in a catheter in using the catheter, and it has input port 45 which is an insertion port of the linear body and input port 46 which is a physiological saline injection port for injecting physiological saline into the catheter.

Measurement device 101 is incorporated in a passage communicating input port 45 and output port 47 with each other within Y-connector 200. Linear body 1 is, for example, a linear medical appliance such as a guide wire and a catheter inserted in a vessel in a body such as a blood vessel and a ureter, or a wire having an embolus coil attached at a tip end for embolizing an aneurysm. Linear body 1 is guided to a destination in the body through an operation from the input port 45 side.

By measuring compressive force and pulling force in the direction of the longitudinal axis applied to linear body 1, reaction force against compressive force and pulling force, that is, load applied by linear body 1 to the vessel in the body, can be measured. Namely, contact of the tip end of the medical appliance with the inner wall of the vessel, linear body 1 being caught in the vessel in the body, or the like can be sensed, and excessive load can be prevented from acting on the vessel in the body.

In addition, since measurement device 101 is incorporated in Y-connector 200, linear body 1 can be operated through input port 45 of Y-connector 200 while a medicine can be injected through input port 46. For example, physiological saline for reducing friction between the catheter and the guide wire can be injected through input port 46. In addition, after the catheter inserted in the blood vessel is guided from the outside of a human body to the destination, a contrast medium can be injected through input port 46 so that the contrast medium can reach the destination in the body.

In inserting a coil and a delivery wire in a catheter in coil embolization, a Y-connector is employed. Therefore, integration of the Y-connector with measurement device 101 or measurement device 102 is very useful because usability so far of the Y-connector remains unchanged. Since the Y-connector is disposable, such a structure that the Y-connector and a sensor housing can be separated from each other may be adopted. In the case of separation, instead of measurement device 101 and measurement device 102, measurement device 103 or measurement device 104 can be employed.

Another embodiment of the present invention will now be described with reference to the drawings. It is noted that the same or corresponding elements in the drawings have the same reference characters allotted and description thereof will not be repeated.

### <Seventh Embodiment>

Fig. 17 is a diagram showing a construction of a training device according to a seventh embodiment of the present invention.

Referring to Fig. 17, a training device 300 includes measurement device 101 or measurement device 102, a guide wire (linear body) 22, a catheter 23, converter 25, a simulator 26, a cable 28, and visualizing instrument 18.

Catheter 23 is connected to measurement device 101, and guide wire 22 that has passed through measurement device 101 is inserted in catheter 23.

When an operator 24 holding guide wire 22 applies compressive force or pulling force to guide wire 22 in order to advance guide wire 22 into simulator 26 or pull out guide wire 22 from simulator 26, that compressive force or pulling force is displayed by means of visualizing instrument 18.

Simulator 26 simulates a human body and displays an image equivalent to a perspective image of a vessel in a human body. Operator 24 in training of a medical device operates guide wire 22 while viewing an image displayed on simulator 26. Simulator 26 varies insertion resistance and pull-out resistance of inserted guide wire 22. Resistance during operation, i.e., compressive force and pulling force applied to guide wire 22 that are measured by measurement device 101 or measurement device 102, is displayed on visualizing instrument 18 and also transmitted to simulator 26 through cable 28. Simulator 26 changes insertion resistance and pull-out resistance of guide wire 22 based on transmitted compressive force and pulling force. When compressive force and pulling force applied to guide wire 22 each exceed a prescribed threshold value, a warning alarm is output from speaker 21.

Converter 25 converts a signal from measurement device 101 in accordance with equipment to be connected, and outputs a resultant signal to visualizing instrument 18 and speaker 21.

According to the construction as above, manipulation of a skilled operator can be quantified and manipulation of a less experienced operator can quickly be improved. In addition, manipulation by the operator can be recorded together with a perspective image, as records during operation.

Though measurement device 101 and simulator 26 are separate from each other in Fig. 17, the construction may be such that measurement device 101 and simulator 26 are integrated. Further, the construction may be such that compressive force applied to guide wire 22 is additionally displayed on a simulated perspective image displayed on simulator 26, instead of visualizing instrument 18.

It should be understood that the embodiments disclosed herein are illustrative and non-restrictive in every respect. The scope of the present invention is defined by the terms of the claims, rather than the description above, and is intended to include any modifications within the scope and meaning equivalent to the terms of the claims.

### REFERENCE SIGNS LIST

1 linear body; 2 main body; 3 through hole; 4A, 4B input/output port; 5A, 5B restraint portion; 6 detection portion; 10 illumination circuit; 12 conversion circuit; 13 linear body selector; 18 visualizing instrument; 19 visualizing instrument; 20 auralizing instrument; 21 speaker; 22 guide wire; 23 catheter; 24 operator; 25 converter; 26 simulator; 28 cable; 29 light source; 30, 53, 54 line sensor; 41 sheath; 42 coil portion; 43 delivery wire portion; 44 sheath groove; 45, 46 input port; 47 output port; 51 housing; 52, 55, 56 marker; 61, 62 boundary portion; 81, 82 inner wall; 83 recess portion; 84 inner wall; 101 to 105 measurement device; 200 connector; and 300 training device.

## Claims

1. A measurement device for measuring compressive force and pulling force in a direction of a longitudinal axis applied to a linear body (1) having flexibility, comprising:
a main body (2) in which a through hole (3) for inserting said linear body (1) is formed, said through hole (3) being formed to allow bending of said linear body (1) in an arc shape in said through hole (3) and variation in degree of bending of said linear body (1) in accordance with said compressive force and said pulling force;
a sensor (30) for detecting a degree of bending of said linear body (1); and
a conversion circuit (12) for converting said degree of bending detected by said sensor (30) into a signal indicating compressive force and pulling force applied to said linear body (1),
said conversion circuit (12) converting detected said degree of bending into a signal indicating compressive force applied to said linear body (1) when the degree of bending of said linear body (1) increases as compared with the degree of bending of said linear body (1) while compressive force and pulling force are not applied to said linear body (1), and converting detected said degree of bending into a signal indicating pulling force applied to said linear body (1) when the degree of bending of said linear body (1) decreases as compared with the degree of bending of said linear body (1) while compressive force and pulling force are not applied to said linear body (1).

2. The measurement device according to claim 1, wherein
said sensor (30) detects a position of said linear body (1) in said through hole (3),and
said conversion circuit (12) converts an amount of displacement from a reference position of said linear body (1) while compressive force and pulling force are not applied to said linear body (1) into a signal indicating compressive force applied to said linear body (1) when said linear body (1) is displaced from the reference position toward an outer periphery, and converts an amount of displacement from said reference position into a signal indicating pulling force applied to said linear body (1) when said linear body (1) is displaced from said reference position toward an inner periphery.

3. The measurement device according to claim 1, wherein
said through hole (3) has
a first end portion (4A) and a second end portion (4B),
a first restraint portion (5A) and a second restraint portion (5B) provided at said first end portion (4) and said second end portion (4B) respectively, in a manner adjacent thereto, for restricting movement of said linear body (1) in a direction other than the direction of the longitudinal axis, and
a detection portion (6) provided between said first restraint portion (5A) and said second restraint portion (5B), formed such that said linear body (1) is bent in an arc shape, and expanded in a direction of an inner periphery and a direction of an outer periphery of said linear body (1) bent in the arc shape.

4. The measurement device according to claim 3, wherein
said through hole (3) further has a groove portion (44) provided between said first restraint portion (5A) and said second restraint portion (5B) and connected to said detection portion (6), and being greater in length in a direction at a right angle to a direction of insertion of said linear body (1) and a direction of radius of said linear body (1) bent in the arc shape than said detection portion (6).

5. The measurement device according to claim 4, wherein
said groove portion (44) is connected to an end portion of said detection portion (6) on an inner periphery side of said linear body (1) bent in the arc shape.

6. The measurement device according to claim 1, wherein
said sensor (30) is attachable to/removable from said main body (2),
said main body (2) is provided with a marker (52), and
said sensor (30) detects a position of said marker (52) while it is attached to said main body (2) and corrects the detected degree of bending of said linear body (1) based on the detected position of said marker (52).

7. The measurement device according to claim 1, wherein
said sensor (30) is an optical line sensor (30) including a light receiver (30) receiving light emitted by a light source (29), for detecting the degree of bending of said linear body (1) by detecting a position at which a quantity of light received by said light receiver (30) decreases as said linear body (1) cuts off the light emitted by said light source (29).

8. The measurement device according to claim 1, comprising a plurality of said sensors (30), wherein
said conversion circuit (12) converts said degree of bending detected by said plurality of sensors (30) into compressive force and pulling force applied to said linear body (1).

9. The measurement device according to claim 1, comprising at least one of a visualizing instrument (18, 19) for displaying compressive force and pulling force resulting from conversion by said conversion circuit (12) and an auralizing instrument (20, 21) for converting compressive force and pulling force resulting from conversion by said conversion circuit (12) into voice and sound.

10. The measurement device according to claim 1, wherein
said linear body (1) is a linear medical appliance for insertion in a vessel in a body.

11. A measurement device for measuring compressive force and pulling force in a direction of a longitudinal axis applied to a linear body (1) having flexibility, comprising:
a main body (2) in which a through hole (3) for inserting said linear body (1) is formed, said through hole (3) being formed to allow bending of said linear body (1) in an arc shape in said through hole (3) and variation in degree of bending of said linear body (1) in accordance with said compressive force and said pulling force;
a sensor (30) for detecting a degree of bending of said linear body (1); and
a conversion circuit (12) for converting said degree of bending detected by said sensor (30) into a signal indicating compressive force and pulling force applied to said linear body (1),
said through hole (3) being formed such that the degree of bending of said linear body (1) increases when compressive force is applied to said linear body (1) as compared with when compressive force and pulling force are not applied to said linear body (1) and such that the degree of bending of said linear body (1) decreases when said pulling force is applied to said linear body (1) as compared with when compressive force and pulling force are not applied to said linear body (1).

12. A medical device comprising the measurement device according to any one of claims 1 to 11.

13. A training device comprising the measurement device according to any one of claims 1 to 11.

14. A measurement method of measuring compressive force and pulling force
in a direction of a longitudinal axis applied to a linear body (1) having flexibility, comprising the steps of:
inserting said linear body (1) into a through hole (3) formed to allow bending of said linear body (1) in an arc shape therein and variation in degree of bending of said linear body (1) in accordance with said compressive force and said pulling force;
detecting a degree of bending of said linear body (1); and
converting detected said degree of bending into a signal indicating compressive force and pulling force applied to said linear body (1) based on prescribed correlation between the degree of bending of said linear body (1) in said through hole (3) and compressive force and pulling force.
